# EUROPEAN PATENT APPLICATION

(11) **EP 1 184 666 A2**
(43) Date of publication of application: **06.03.2002**
(21) Application number: 01119886.8
(22) Date of filing: 17.08.2001
(51) Int. Cl.: G01N 33/543

(54) **Auxiliary haptens and haptenylated agents in displacement immunoassays**

(30) Priority: 21.08.2000 EP 00117916
(71) Applicant: Roche Diagnostics GmbH, 68305 Mannheim (DE); F. HOFFMANN-LA ROCHE AG, 4070 Basel (CH)
(72) Inventor: Wild, Thomas, Dr., 82377 Penzberg (DE); Huber, Erasmus, Dr., 86923 Finning (DE); Karl, Johann, Dr., 82380 Peissenberg (DE); Sluka, Peter, Dr., 82362 Weilheim (DE); Stegmueller, Peter, 86199 Augsburg (DE); Wild, Norbert, Dr., 82538 Geretsried/Gelting (DE)

(57) **Abstract**

The present invention relates to methods for detection or measurement of an analyte in a sample by a binding assay comprising at least one hapten/anti-hapten or hapten-like binding pair. More specifically, the invention is directed to processes and test kits comprising the hapten or hapten-like molecule of such binding pair in free form.

## Description

The present invention relates to methods for detection or measurement of an analyte in a sample by a binding assay comprising at least one hapten/anti-hapten or hapten-like binding pair. More specifically, the invention is directed to processes and test kits comprising the hapten or hapten-like molecule of such binding pair in free form.

Binding assays nowadays are widely used in research settings as well as in clinical diagnostic environments. The use of binding assays dates back about 30 years (Engvall E. and Perlmann P. (1971), Immunochemistry 8, 871; van Weemen, B.K. and Schuures A.H.W.M. (1971), FEBS letters 15, 232). Since then enormous progress has been made and methods for carrying out specific binding assays as well as practical applications thereof have become general knowledge to the skilled artisan. Methods and procedures summarized in related text books are herewith included by reference and only few examples shall be specifically mentioned: "Practice and theory of enzyme immunoassays" by P. Tijssen (1992) Elsevier Science Publishers B.V.; and various editions of "Methods in Enzymology", Colowick S.P., Caplan N.O., Eds., Academic Press, dealing with immunological detection methods.

Binding assays are usually classified according to the mode of detection used, i.e., as enzyme immunoassays, radio immunoassays, fluorescence polarization immunoassays, chemiluminescence immunoassays, turbidimetric assays, etc.. On the other hand binding assays may be grouped according to the assay procedures used. Well known examples of such procedures are competitive assay formats, sandwich-type assay formats as well as assays based on precipitation or agglutination principles.

Most binding assays require that at least one member of a specific binding pair is either directly or indirectly coated or attached to a carrier. In many cases such carrier is a solid phase material, e.g., like a microtiter plate, a latex or glass particle, or a test strip to mention only the most prominent ones. It is a well known phenomenon that different bio-molecules may exhibit quite different physico-chemical properties. Such different properties may result in quite different requirements for attachment or coating of such molecules to a carrier material.

With tremendous advantage hapten or hapten-like binding pairs have been used in order to indirectly bind members of a specific binding pair to carrier. The best known examples of a hapten-like, non-immunological, binding pair is the biotin/avidin or biotin/streptavidinsystem. Properties and advantages of biotin-(strept)avidin-systems are e.g. described or disclosed in U.S. Patent No. 4,298,685 (Parikh, et al.); Green, Methods in Enzymology (1990) 184:51-67; Green et al., Biochem. J. (1971) 125:781-791; Weber et al., Science (1989) 243:85-88; Morgan et al., Polym. Sci., Part A: Polm. Chem. (1994) 32:1331; Akiyama, Protein Expression Purif. (1992) 3:427; Nutikka Clin. Biochem. (1991) 24:469-473.

US 6,096,508 relates to problems encountered due to background signals caused by non-specific binding in nucleic acid hybridization-based assays using nylon membranes which rely on the biotin-avidin/streptavidin interaction for detection. Reduction of back-ground signal is observed in a heterogeneous assay set-up, which includes one or several washing steps. Free biotin is added to one or several washing buffers.

A full range of products, as commercialized by Roche Diagnostics GmbH under the brand names Enzymun® and ELECSYS® , is based on the tremendous advantages brought about by the streptavidin/ biotin system.

Amongst other criteria assay sensitivity and assay precision are very important quality criteria for binding assays.

It was the task of the present invention to investigate whether it is possible to improve assay methods which are based on the use of a hapten or hapten-like binding pair in terms of sensitivity and/or precision in order to provide improved assay methods and test kits.

It has surprisingly been found that it is possible to improve methods for detection or measurement of an analyte in a sample which are based on a specific binding assay comprising at least one hapten/anti-hapten or hapten-like binding pair, by addition to or presence of the hapten or hapten-like molecule in its free form in at least one incubation step.

### Summary of the invention:

The invention is based on the positive and surprising effects caused by the addition to or presence of free hapten or hapten-like molecules in a binding assay making use of such hapten or hapten-like binding pair.

It specifically has been found and demonstrated, that addition to or presence of free hapten or hapten-like molecule in at least one reaction mixture or incubation step results in an improved method for detection or measurement of an analyte. The improved methods yield assays which show a better sensitivity and/or assay precision.

In a preferred embodiments the present invention relates to the advantageous use of free hapten or hapten-like molecules in improved assay procedures.

Test kits to perform an assay based on the use of hapten/anti-hapten or hapten-like binding pairs for detection or measurement of an analyte in a sample which comprise at least one reagent containing said hapten or hapten-like molecule in free form are also disclosed and represent a further embodiment of the present invention.

### Detailed description of the invention:

In a first embodiment the present invention relates to a method for detection or measurement of an analyte in a sample by a specific binding assay comprising at least one hapten/anti-hapten or hapten-like binding pair, characterized in that, in at least one incubation step said hapten or said hapten-like molecule in free form is present.

Whereas some analytes, e.g., enzymes with specific activity may be detected or measured directly, for example with clinical/chemical procedures, many, many other analytes are detected or measured in methods employing a binding partner for such analyte. Analyte in the sense of the present invention may be any molecule which is capable of binding to a specific binding partner, e.g., an antibody, a nucleic acid, a lectin, a receptor, etc.. In certain cases, in assays designed to measure such analytes, it may be preferable to use analyte analogues, which can mimic properties of the analyte investigated, e.g., a small peptidic sequence, representing a single epitope of a larger molecule. Such epitopes in immunological terms may represent haptens. In order to make a clear distinction, it has to be understood that such analyte-related haptens, which directly participate in binding of an analyte specific binding partner, are not within the scope of the present invention.

Preferred analytes according to the present invention comprise peptides, proteins, modified proteins, polysaccharides, nucleic acids, hormones, as well as small molecules like drugs, metabolites, and the like. Representative analytes, include but are not limited to alkaloids such as morphine, codeine, heroin, dextromethorphan, their derivatives and metabolites; cocaine alkaloids, which include cocaine and benzyl ecgonine, their derivatives and metabolites; drugs derived from marijuana, which includes cannabinol and tetrahydrocannabinol; aminoglycosides, such as gentamycin, kanamicin, tobramycin, amikacin, and so forth.

Such binding assays preferably are designed to specifically detect one or few analyte molecules under investigation.

Binding assays usually are based on at least one binding pair, comprising the analyte, or an analogue thereof, and a binding partner for said analyte. Theoretical and practical members of such binding pairs are well known to the skilled artisan, most prominent being the use of nucleic acid binding pairs, as well as binding pairs like sugar/lectin, hapten/antibody or antigen/antibody.

Binding assays based on the use of antibodies are broadly used and represent a preferred embodiment of the present invention. Binding assays based on used immunological reagents usually are classified according to the mode of detection or according to the assay procedure used. A further distinction is made depending on whether washing steps are included into the procedure (so-called heterogeneous assays) or whether reaction and detection is performed without washing step (so-called homogeneous assays). All the essential terms, procedures and devices are known to the skilled artisan from text books in the field, e.g., Tijssen, supra, and "Methods in Enzymology", supra, are herewith included by reference.

Haptens are small molecules which themselves are not immunogenic. However, when conjugated to appropriate carrier molecules, such conjugates may be used to render them immunogenic and to thus generate an appropriate immune response. Polyclonal as well as monoclonal antibodies against haptens of various kinds are well known in the art. Well known haptens are for example fluoresceine di-nitro-phenyl-phosphate (DNP), steroidal hormones, many low molecular weight drugs, digoxin and digoxigenin.

The term hapten-like molecules is used to describe small molecules which are part of a non-immunological binding pair. Well known examples of such binding pairs are amongst others the prosthetic groups of enzymes and the enzyme, substrate derivatives and corresponding enzymes, biotin/avidin, or biotin/streptavidin. Preferably such hapten-like molecules are characterized by a molecular weight of less than 10 kD, more preferred less than 5 kD, more preferred less than 2 kD and most preferred less than 1 kD. Binding pairs comprising a hapten or hapten-like molecule and exhibiting rather high affinity are preferred. Preferably such affinity is at least 10 ⁻⁹ M/l, more preferred 10 ⁻¹⁰ M/l, most preferred 10 ⁻¹¹ M/l or higher.

The hapten or hapten-like molecule in the sense of the present invention are not related to the analyte under investigation. Rather, they are used to indirectly mediate the binding between at least one analyte specific component of the binding assay (e.g. an antibody binding to an analyte, or an analyte binding to a receptor, e.g. an antibody) and a more generally applicable assay component. Such more generally applicable assay components comprise e.g. a labeled detection reagent (e.g. labeled anti-hapten antibodies), or a solid phase or carrier material coated with a binding partner for said hapten or hapten-like molecule.

Many assay set-ups are known from the art wherein at least one analyte specific component is coupled or linked to a hapten or a hapten-like molecule, e.g. biotinylated or digoxigenylated antibodies, etc. In a more generic manner the term haptenylated analyte specific component is used to indicate that a hapten or a hapten-like molecule is covalent linked to an analyte specific component.

A binding pair based on a hapten or a hapten-like molecule may be used for various purposes when carrying out a specific binding assay relying on a method based on such a binding pair. Preferably hapten or hapten-like binding pairs are used to attach a detectable label to one of the components of such a binding assay, e.g., to the analyte or to an antibody thereto. Most preferably a hapten or hapten-like binding pair is used to indirectly attach at least one of the components of the binding assay onto a solid phase or carrier, comprising a binding partner for said hapten or hapten-like molecule. Solid phase materials comprise, amongst others, the surfaces of microtiter plates, tubes, or beads of various polymeric nature, like for example glass, latex or plastic materials, membranes as used in dry chemistry devices, e.g., for preparing test strips. Carrier materials comprise amongst others polymeric bio-molecules for example carbohydrates, (synthetic) polypeptides, nucleic acids and nucleic acid derivatives.

In order to carry out a binding assay - this is true also for assays comprising a hapten/anti-hapten or hapten-like binding pair - it is necessary to mix sample and reagent(s), resulting in one or several reaction mixtures. The sample containing the analyte and the reagents used to carry out the binding assay are incubated sequentially or simultaneously. It has now been found to be advantageous if free hapten or free hapten-like molecule is present in at least one incubation step in an assay comprising the respective binding pair, i. e. the haptenylated analyte specific component and a binding partner for said hapten or said hapten-like molecule.

Free hapten or free hapten-like molecule shall be understood to comprise the hapten or hapten-like molecule itself as well as derivates (e. g.) conjugates, or analogues thereof, which may be used essentially analogous to the free molecules. Analogues are molecules of comparable molecular weight exhibiting essentially the same binding properties as the corresponding free hapten or hapten-like molecule and comprising minor modifications not significantly influencing the binding to their respective binding partner. In a preferred embodiment the non-derivatized hapten or hapten-like molecule is used.

Preferred derivatives and preferred analogues comprise the binding domain of the hapten or hapten-like molecule but may be modified as desired at those parts of the molecule not involved into the binding to the respective and appropriate analogue binding partner. Appropriate derivatives can be easily identified by their ability to improve an immunoassay as described in the present invention. In case biotin is used as the hapten-like molecule a preferred analogues of biotin are biocytin and biotin-methyl ester, the most preferred analogue of biotin is biocytin. It is preferred to use an analogue of a hapten or a of a hapten-like molecule in a method according to the present invention.

The free or free hapten-like molecule may be added as a separate component to the reaction mixture or may be present in one of the reagent combinations used to carry out the assay. It is preferred to carry out the assay in such ways that free hapten or free hapten-like molecule is added to or present in the reaction mixture before the haptenylated analyte specific component is present and the incubation step is performed. The at least one incubation step therefore is characterized by the simultaneous presence of a haptenylated analyte specific component, a binding partner for the hapten or hapten-like molecule comprised therein, and the free hapten or hapten-like molecule. Incubation steps usually last from a few minutes up to a few hours.

It has been demonstrated that digoxin or digoxigenin may be used with great advantages as a general means for binding and/or detection of other bio-molecules (US-5,344,757). Other bio-molecules, for example antibodies, antigens or nucleic acids, are conjugated (haptenylated) with digoxin or digoxigenin and antibodies to digoxin or digoxigenin are used to indirectly bind the haptenylated molecule to a carrier or solid phase or to detect such a haptenylated component. In a preferred embodiment according to the present invention the hapten/anti-hapten system comprises digoxin or digoxigenin and anti-digoxin or anti-digoxigenin antibodies, respectively.

The best known examples of hapten-like non-immunological binding pairs are the biotin/avidin or the biotin/streptavidin system. Reference to some basic literature is given above, the procedures described in U.S. Patent No. 4,298,685 (Parikh, et al.); Green, Methods in Enzymology (1990) 184:51-67; Green et al., Biochem. J. (1971) 125:781-791; Weber et al., Science (1989)243:85-88; Morgan et al., Polym. Sci., Part A: Polm. Chem. (1994) 32:1331; Akiyama, Protein Expression Purif. (1992) 3:427; Nutikka Clin. Biochem. (1991) 24:469-473., herewith included by reference. In a preferred embodiment of the present invention the hapten-like binding pair comprises biotin and avidin or/and streptavidin. Preferably the avidin or streptavidin component is bound to a carrier or solid phase material and used to indirectly bind a biotinylated component of the binding assay carried out.

In a further embodiment the invention is used in a method to improve the sensitivity of a specific binding assay used in the detection or measurement of an analyte in a sample comprising at least one hapten/anti-hapten or hapten-like binding pair, characterized in that, in at least one incubation step said hapten or said hapten-like molecule in free form is present.

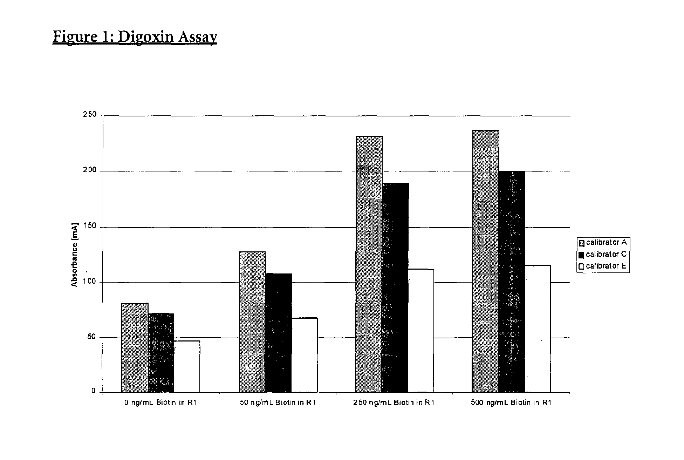

Sensitivity as used here relates to the minimal amount of analyte which can be detected in the assay procedure applied. The less analyte required the more sensitive the assay. Assay sensitivity in general is correlated to the dynamic range of an assay. Such comparisons from assay to assay preferably are performed using only one modification to the assay per experiment and leaving all other components and procedures essentially unchanged. This way, essentially comparable assays are performed and relevant results are obtained. As demonstrated in Figure 1, the addition of free biotin in an assay for digoxin as described in detail in Example 1, clearly improves the sensitivity of this assay, although all other components and procedures are kept constant.

It has been found that improvements in sensitivity are more pronounced when using rather small analytes. It is preferred to use the inventive methods and procedures aiming at improved sensitivity with analytes of less than 10 kD molecular weight, more preferred 5 kD and most preferred less than 2 kD molecular weight. Such analytes especially comprise digoxin, digoxigenin, small molecular weight drugs, steroidal hormones and drugs of abuse.

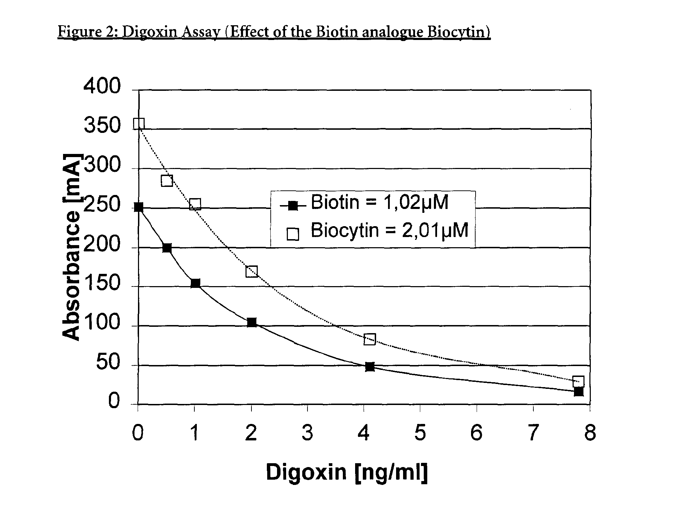

The agglutination reaction, i.e., the assay sensitivity preferably is further modulated and optimized by use of an analogue of a hapten or a hapten-like molecule, e.g. by use of biocytin if the analyte specific component is biotinylated. Such improvements are demonstrated in Example 2 and shown in Figure 2.

In a further embodiment the invention as disclosed is used in a method to improve the precision of a specific binding assay used in the detection or measurement of an analyte in a sample comprising at least one hapten/anti-hapten or hapten-like binding pair, characterized in that, in at least one incubation step said hapten or said hapten-like molecule in free form is present.

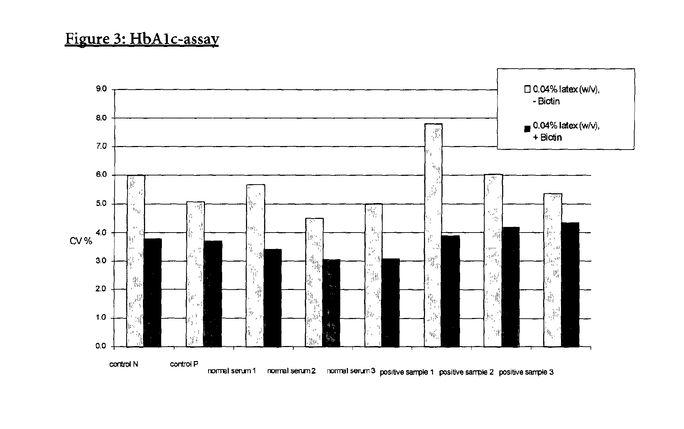

Precision is used to refer to the calculated result with standard deviation. The lower the standard deviation or co-efficient of variation (CV) the better the assay precision. As shown in Figure 3, the use of free hapten or free hapten-like molecule according to a method of the present invention, as described in Example 3 has led to an improved assay precision.

Heterogeneous immunoassays incorporate washing steps, such washing steps having the great advantage that unbound and/or potential interfering substances may be washed efficiently off. This probably is the major reason, why heterogeneous assays in many cases can be designed to be more sensitive as compared to homogeneous assays. It has now surprisingly been found that it is possible to significantly improve homogeneous immunoassays by applying a method as disclosed.

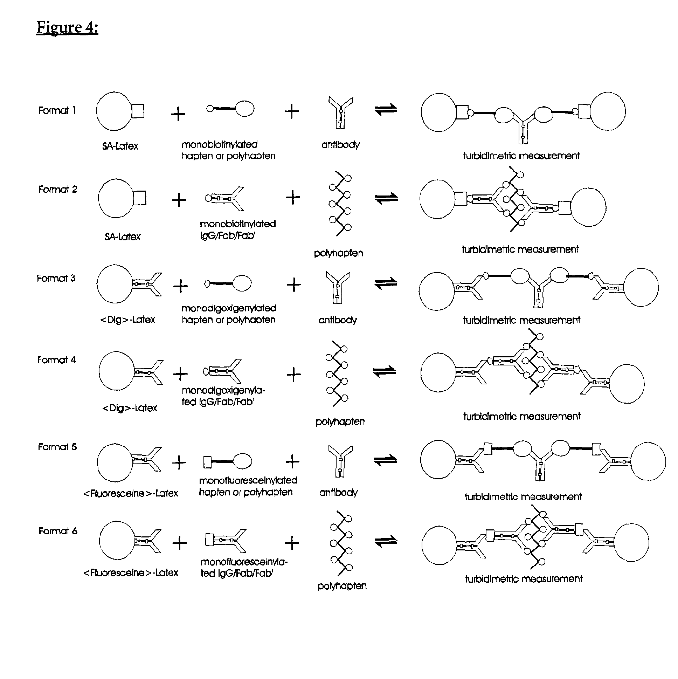

Therefore, in a preferred embodiment the present invention relates to a method for detection or measurement of an analyte in a sample by a homogeneous immunoassay comprising at least one hapten/anti-hapten or hapten-like binding pair, characterized in that, in at least one incubation step said hapten or said hapten-like molecule in free form is present. Typical examples of homogeneous immunoassays making use of coated (latex) particles are summarized in Figure 4 to further illustrate some preferred assay principles.

Homogeneous immunoassays according to the present invention are preferably based on the principles of agglutination. Such assays preferably are set up to detect or measure an analyte either due to increased or due to decreased agglutination. Such agglutination usually is measured by turbidimetric devices. Preferably particles are used and included in such assay methods, because particle based assays have been found to be more sensitive.

Most preferably latex particles are used. In a preferred embodiment according to the present invention the assay method is of the homogeneous type and further characterized in that coated latex particles are used in said method.

Latex particles to be used in the above described homogeneous immunoassays may e.g., be coated with antibodies to a hapten, e.g. antibodies, against digoxin or digoxigenin or they may be coated with avidin or streptavidin. Most preferably the coating with avidin or streptavidin is performed according to procedures as described in US 5,268,306. It represents a preferred embodiment of the present invention to use free biotin or analogues thereof in a homogeneous assay procedure based on use of latex particles coated with streptavidin.

As discussed above, the skilled artisan based on the state of the art knowledge and procedures will have no problems requiring own inventive efforts or undue burden to him, to set up a binding assay employing a hapten or hapten-like binding pair. The following discussion is meant to further illustrate the invention but not to limit it to specific examples given.

In case of homogeneous assays it is preferred to reduce the number of reagents required as much as possible. For most analytes, e.g. as detected on the Roche/Hitachi analyzer series it has been possible to reduce and limit the number of reagents to two. Reagent R1 may for example comprise a buffer salt, sodium chloride, bovine serum albumin, a preservative, a detergent, the biotinylated component and an optimized amount of free biotin. Reagent R2 in most cases is designed to comprise a buffer salt, bovine serum albumin, a preservative and the streptavidin-latex with an optimized binding capacity for biotin. The amount of free biotin added is optimized with respect to the binding capacity of the streptavidin latex in R2 and concentration of the biotinylated component in R1. Such optimization is performed according to routine procedures known in the art.

Various buffers and additives may be used for Reagent 1 and Reagent 2 to achieve the desired pH and the stability of the reagents. Illustrative buffers include 2-(N-Morpholino)ethanesulfonic acid, N-(2-Hydroxyethyl)piperazine-N'-(2-ethanesulfonic acid), (Piperazine-N,N'-bis[2-ethanesulfonic acid / 1,4-Piperazinediethanesulfonic acid) and Tris(hydroxymethyl)aminomethane. In an individual assay one or another buffer may be preferred. The same is true for special additives like sodium chloride, bovine serum albumin, detergents like Triton X-100 and preservatives like NaN3. Moderate temperatures usually are employed for carrying out a homogeneous assay. Incubation temperatures will normally range from 5 to 35°C, more usually from 20 to 25°C. The concentration of analyte to be assayed will generally vary from 10⁻⁵ to 10⁻¹³ M, more usually from 10⁻⁷ to 10⁻¹¹ M.

Assay requirements depend e.g. on whether the assay shall be qualitative, semi-quantitative or quantitative, and the mode of detection (turbidimetric or nephelometric) used.

The sensitivity required is usually defined to match the concentrations of analyte routinely found or expected in the samples to be analyzed. Assays shall be sensitive enough to ensure that a biologically and/or diagnostically significant variation in analyte concentration results in a significant and measurable signal difference according to the procedure used.

The final concentration of free hapten or hapten-like molecule will vary very much depending on the kind of binding assay used. Especially the concentration in relation to the haptenylated compound as used in the assay has to be determined carefully. Amounts or concentrations of free hapten or hapten-like molecule are preferred which lead to assay improvements in term of sensitivity and/or precision. In case of homogeneous assays wherein the haptenylated compound is used in a concentration of about 0.6 - 285 nM it is preferred to use 20 - 210 nM of free biotin in reagent R1 and a streptavidin load of 280-420 nM on the surface of the latex microparticles in reagent R2.

According to the present invention use of free hapten or of free hapten-like molecules in an improved homogeneous assay method for detection or measurement of an analyte in a sample said detection or measurement being based on a binding assay comprising said hapten or hapten-like molecule as part of a specific binding pair also represents a preferred embodiment.

Whereas the presence of free hapten or hapten-like molecule may be advantageous in various steps of carrying out the inventive procedure it is preferred that said free hapten or hapten-like molecule is present in the reaction mixture before the haptenylated assay component is added to said reaction mixture.

It is standard practice to provide test kits to customers, comprising most if not all reagents necessary to carry out a method of detection or measurement of an analyte. It is of great advantage to include into such a test kit an additional bottle comprising free hapten or free hapten-like analogue. It is also possible to include such free hapten or hapten-like molecule in at least one of the other reagent bottles, e.g. the ones comprising at least one of the assay components, preferably the haptenylated component, required to detect or measure an analyte. Further preferred is therefore a test kit to perform an assay for detection or measurement of an analyte in a sample making use of a hapten/anti-hapten or hapten-like binding pair comprising at least one reagent containing said hapten or hapten-like molecule in free form.

The following examples, references, sequence listing and figures are provided to aid the understanding of the present invention, the true scope of which is set forth in the appended claims. It is understood that modifications can be made in the procedures set forth without departing from the spirit of the invention.

### Description of the Figures

- **Figure 1**: **Measuring range of different digoxin assays**

Signals generated dependent on the concentration of biotin in the digoxin assay reagent are given. It is obvious that the measuring range correlated to the signal generated increases, if free biotin is added to reagent R1.
- **Figure 2**: **Modulation of specific agglutination by the biotin analogue biocytin.**

The agglutination reaction is further modulated and improved by the use of biocytin. The concentrations of biotin and biocytin have been titrated to give optimal agglutination signals. Biocytin clearly generates higher signals and a better differentiation of samples.
- **Figure 3**: **Precision of different Hb**_{**A1c**} **assays**

This figure shows the Hb_{A1c} assay precision [in % CV; CV = coefficient of variation] in presence and absence of free biotin in reagent R1.
- **Figure 4**: **Overview of preferred assay formats**

Figure 1 gives a summary of the preferred assay formats of the present invention. The symbols <...> are used to indicate that an antibody directed against the antigen in brackets is used. Abbreviations are as follows:
- SA =: streptavidin
- IgG =: immunoglobulin of the γ-type
- Fab and Fab' =: represents fragments of immunoglobulin as obtained by enzymatic cleavage according to state of the art procedures.

### Example 1: Assay for Digoxin

### Materials and Equipment

### Reagent R1

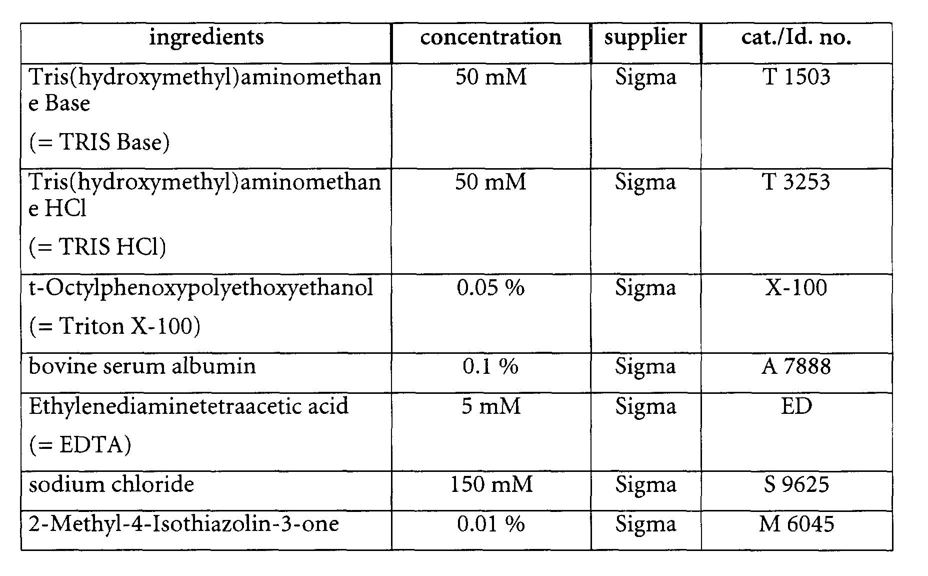

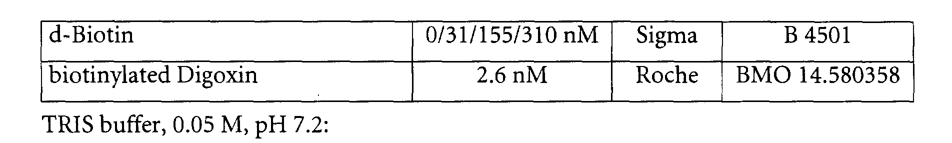

7.02 g of Tris(hydroxymethyl)aminomethane HCl dissolved in 1.0 L of water combined with 0.67 g Tris(hydroxymethyl)aminomethane Base gives at 25°C a pH of 7.2

Hapten-biotin conjugates have been synthesized according to Huber, et al., U.S. Patent No. 5,219,764.

### Reagent R2

| **ingredients** | **concentration** | **supplier** | **cat./Id. no.** |
|---|---|---|---|
| N-(2-Hydroxyethyl)piperazine-N'-(2-ethanesulfonic acid) | 50 mM | Sigma | H 7523 |
| (= HEPES) | | | |
| t-Octylphenoxypolyethoxyethanol | 0.01 % | Sigma | X-100 |
| (= Triton X-100) | | | |
| bovine serum albumin | 1 % | Sigma | A 7888 |
| 2-Methyl-4-Isothiazolin-3-one | 0.01 % | Sigma | M 6045 |
| anti-Digoxin antibody (monoclonal antibody) | 0.2 nM | Roche | 2157861990 |
| streptavidin-latex (127 nm particles, carboxylate-modified) | 0.05 % [s/v]* | Seradyn | lot 640CJ |

| | | | |
|---|---|---|---|
| * [s / v] = solids per volume | | | |

HEPES (pKa = 7.5 at 25°C) buffer, 0.05 M, pH 7.5:

11.9 g of N-(2-Hydroxyethyl)piperazine-N'-(2-ethanesulfonic acid) dissolved in 1.0 L of water

All assay measurements have been done on a Hitachi 917 Automatic Analyzer, Part No. 714-0016, Serial No. 1004-8, from Hitachi, Ltd. Tokyo, Japan, according to the following assay protocol:

| **step** | **pipetting of** | **volume [µL]** | **Hitachi measuring points at 546 nm** |
|---|---|---|---|
| 1 | R1 | 150 | 1 - 17 |
| | Sample | 20 | |
| 2 | R2 | 150 | 18-33 |

The sample material is human serum or human plasma (e.g. heparin-, EDTA-, citrateplasma).

Results as obtained with calibrators containing 0, 1.0 and 4.1 ng/mL digoxin, respectively, are given in Figure 1.

The results of a typical experiment with clinical samples were as follows (Table 1):

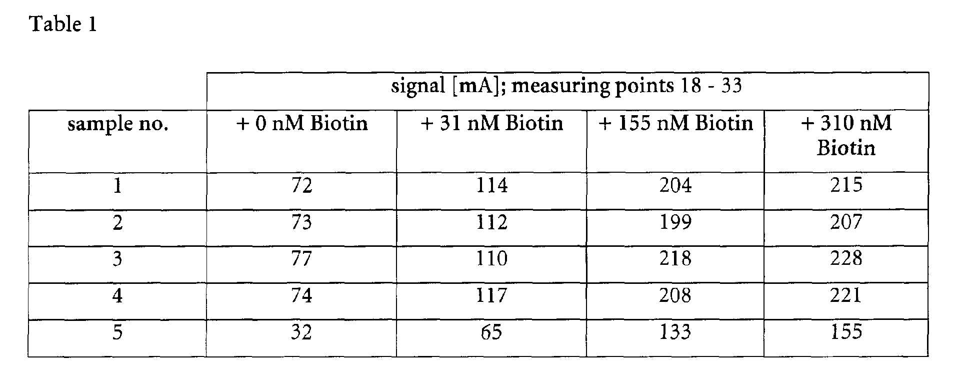

It is obvious that addition of free biotin leads to a pronounced increase in signal (absorbance measured as mA).

### Example 2: Assay for Digoxin: Effect of the biotin analogue biocytin Materials and Equipment

### Reagent R1

| ingredients | concentration | supplier | cat./Id. no. |
|---|---|---|---|
| 2-(n-Morpholino)-ethansulfonic acid (= MES) | 50 mM | Sigma | M-2933 |
| sodium chloride | 150 mM | Sigma | S-9625 |
| polyoxyethylenesorbitan (Tween-20) | 0.4 % | Sigma | P-7949 |
| bovine serum albumin | 0.1 % | Sigma | A-7888 |
| dextran sulfate | 2.0 % | Sigma | D-6001 |
| 2-Methyl-4-Isothiazolin-3-one | 0.01 % | Sigma | M-6045 |
| 2-Chlor-acetamid | 0.25 % | Fluka | 22788 |
| biotinylated Digoxin | 19.4 nM | Roche | BMO22.250123 |
| d-biotin | 0.41 - 1.23 µM | Sigma | B-4501 |
| respectively | | | |
| biocytin | 0.81 - 2.42 µM | Sigma | B-1758 |
| MES buffer, 0.05 M, pH 7.0 (adjusted with NaOH) | | | |

Hapten-biotin conjugates have been synthesized according to U.S. Patent No. 5,219,764 (Huber et al.)

### Reagent R2

| ingredients | concentration | supplier | cat./Id. no. |
|---|---|---|---|
| N-(2-Hydroxyethyl)piperazine-N'-(2-ethansulfonic acid (= HEPES) | 5 mM | Sigma | H-7523 |
| bovine serum albumin | 1.0 % | Sigma | A-7888 |
| 2-Methyl-4-Isothiazolin-3-one | 0.01 % | Sigma | M-6045 |
| 2-Chlor-acetamid | 0.25 % | Fluka | 22788 |
| anti-digoxin antibody (monoclonal) | 0.67 nM | Roche | 2157861990 |
| streptavidin-latex (214 nm) | 0.06 % [s/v]* | Poly-Micro-spheres | custom synthesis |

| | | | |
|---|---|---|---|
| * [s/v] = solids per volume HEPES buffer, 0.005M, pH 7.5 (adjusted with NaOH) | | | |

All assay measurements have been done on a Hitachi 717 automatic analyzer, part No 717-0016, serial No. 6100-05, from Hitachi, Ltd. Tokyo, Japan, according to the following assay protocol:

| Step | Pipetting of | Volume [µl] | Hitachi meassuring points at 700 nm |
|---|---|---|---|
| 1 | R1 | 105 | 1 - 23 |
| | Sample | 14 | |
| 2 | R2 | 105 | 24-50 |

The sample material is human serum or human plasma (e.g. heparin-, EDTA-, citrate-plasma).

The biotin and biocytin concentrations added to buffer R1 were titrated within the given range in order to determine the concentration for optimal assay performance. For biotin this concentration was 1.02 µM (= 250 ng/ml) while with biocytin 2.01 µM (750 ng/ml) gave optimal agglutination signals. The results of a typical experiment with an optimized biotin or biocytin concentration are given in table 2 and figure 2.

**Table 2**

| | Signal [mA]; measuring points 28 - 50 | |
|---|---|---|
| Sample No | + 1.02 µM biotin | + 2.01 µM biocytin |
| 1 | 251 | 357 |
| 2 | 200 | 285 |
| 3 | 154 | 255 |
| 4 | 105 | 169 |
| 5 | 49 | 83 |
| 6 | 16 | 29 |

### Example 3: Assay for Hemoglobin A1c

### Materials and Equipment

### Reagent R1

| **ingredients** | **concentration** | **supplier** | **cat./Id. no.** |
|---|---|---|---|
| 2-(n-Morpholino)-ethansulfonic acid (= MES) | 25 mM | Roche | 223794 |
| pH 6.2 | | | |
| Tris(hydroxymethyl)aminomethane HCl | 17 mM | Roche | 708968 |
| (= TRIS HCl) | | | |
| Thesit | 0.5 % | Roche | 1718894 |
| bovine serum albumin | 0.1 % | Roche | 1726544 |
| Ethylenediaminetetraacetic acid | 5 mM | Roche | 808245 |
| (= EDTA) | | | |
| 2-Methyl-4-Isothiazolin-3-one | 0.01 % | Roche | 1085905 |
| d-Biotin | 50 ng/mL | Sigma | B 4501 |
| biotinylated Hemoglobin A1c | 0.1 µg/mL | Roche | BMO 22.620269 |
| Hapten-biotin conjugates have been synthesized according to U.S. Patent No. 5,219,764 (Huber, et al.). | | | |

### Reagent R2

| **ingredients** | **concentration** | **supplier** | **cat./Id. no.** |
|---|---|---|---|
| N-(2-Hydroxyethyl)piperazine-N'-(2-ethanesulfonic acid) (= HEPES) | 5 mM | Roche | 737151 |
| bovine serum albumin | 1 % | Roche | 1726544 |
| 2-Methyl-4-Isothiazolin-3-one | 0.01 % | Roche | 1085905 |
| anti-Hemoglobin A1c antibody (polyclonal antibody) | 40 µg/mL | Roche | 1520458001 |
| streptavidin-latex (127 nm particles, carboxylate-modified) | 0.2%[s/v]* | Seradyn | lot 640CJ |

| | | | |
|---|---|---|---|
| *[s / v] = solids per volume HEPES (pKa = 7.5 at 25°C) buffer, 0.005 M, pH 7.5: 1.19 g of N-(2-Hydroxyethyl)piperazine-N'-(2-ethanesulfonic acid) dissolved in 1.0 L of water | | | |

All assay measurements have been done on a Hitachi 717 Automatic Analyzer, Part No. 717-0016, Serial No. 6100-05, from Hitachi, Ltd. Tokyo, Japan, according to the following assay protocol:

| **step** | **pipetting of** | **volume [µL]** | **Hitachi measuring points at 546 nm** |
|---|---|---|---|
| 1 | R1 | 200 | 1 - 27 |
| | Sample | 2 | |
| 2 | R2 | 50 | 28 - 50 |

The hemolyzed human blood samples were prepared according to Karl J. et al.: Development and Standardization of a New Immunoturbidimetric HbA1c Assay. Klin Lab 39, 991-996 (1993).

The results of a first typical experiment conducted according to this Example are shown in Figure 3, the results of a second typical experiment were as follows (Table 3):

**Table 3**

| **sample no.** | **R1 minus biotin: CV [%]; n = 21** | **R1 plus biotin: CV [%]; n = 21** |
|---|---|---|
| Control N | 6.0 | 3.8 |
| Control P | 5.1 | 3.7 |
| Normal serum 1 | 5.7 | 3.4 |
| Normal serum 2 | 4.5 | 3.0 |
| Normal serum 3 | 5.0 | 3.1 |
| Positive sample 1 | 7.8 | 3.9 |
| Positive sample 2 | 6.0 | 4.2 |
| Positive sample 3 | 5.3 | 4.3 |

It is obvious that the presence of biotin in the above method leads to improved assay precision as is obvious from the reduced CV values found.

### List of References

Engvall, E. and Perlman, P., Immunochemistry 8 (1971) 871-4
Green, N. M., Methods Enzymol 184 (1990) 51-67
Green, N. M., et al., Biochem J 125 (1971) 781-91
Weber, P. C., et al., Science 243 (1989) 85-8
Akiyama, N., et al., Protein Expr Purif 3 (1992) 427-33
Nutikka, A., et al., Clin Biochem 24 (1991) 469-73
Morgan, H., et al. Polymerization of avidin and streptavidin with aromatic bisbiotin ligands in "Polym. Sci., Part A: Polm. Chem." (1994) 1331-1340, Univ. Wales, Bangor/Gwynedd, LL57 1, UK, Inst. Mol. Biomol. Electron.
van Weemen, B. K. and Schuurs, A. H. W. M., FEBS Letters 15 (1971) 232-236
Karl, J., et al., Klinisches Labor 39 (1993) 991-996
US 4,298,685
US 6,096,508
US 5,344,757
US 5,268,306

## Claims

1. Method for detection or measurement of an analyte in a sample by a specific binding assay comprising at least one hapten/anti-hapten or hapten-like binding pair, **characterized in that**, in at least one incubation step said hapten or said hapten-like molecule in free form is present.

2. Method according to claim 1, further **characterized in that**, said hapten/anti-hapten system comprises digoxin or digoxigenin and anti-digoxin or anti-digoxigenin antibodies, respectively.

3. Method according to claim 1 or 2 further **characterized in that**, said hapten-like binding pair comprises biotin and avidin or/and streptavidin.

4. Method according to any of claims 1 to 3 further **characterized in that**, said hapten-like binding pair comprises biocytin and avidin or/and streptavidin.

5. Method to improve the sensitivity of a specific binding assay used in the detection or measurement of an analyte in a sample comprising at least one hapten/anti-hapten or hapten-like binding pair, **characterized in that**, in at least one incubation step said hapten or said hapten-like molecule in free form is present.

6. Method to improve the precision of a specific binding assay used in the detection or measurement of an analyte in a sample comprising at least one hapten/anti-hapten or hapten-like binding pair, **characterized in that**, in at least one incubation step said hapten or said hapten-like molecule in free form is present.

7. Method according to any of claims 1 to 6, further **characterized in that**, said method is a homogeneous immunoassay.

8. Method according to any of claims 1 to 7, further **characterized in that**, coated latex particles are used in said method.

9. Method according to any of claims 1 to 7, further **characterized in that**, said free hapten or hapten-like molecule is present in the reaction mixture before the haptenylated assay component is added to said reaction mixture.

10. Assay kit to perform an assay for detection or measurement of an analyte in a sample making use of a hapten/anti-hapten or hapten-like binding pair comprising at least one reagent containing said hapten or hapten-like molecule in free form.
